# EUROPEAN PATENT APPLICATION

(11) **EP 3 970 774 A1**
(43) Date of publication of application: **23.03.2022**
(21) Application number: 21193348.6
(22) Date of filing: 26.08.2021
(51) Int. Cl.: A61M 25/00

(54) **RIBBON EXTRUSION SEGMENTS FOR CATHETER CONSTRUCTION**

(30) Priority: 28.08.2020 US 202017006024
(71) Applicant: DePuy Synthes Products, Inc., Raynham, MA 02767 (US)
(72) Inventor: PEDROSO, Pedro, Raynham, 02767 (US); HANNA, Chadwin, Raynham, 02767 (US)
(74) Representative: Carpmaels & Ransford LLP

(57) **Abstract**

A highly flexible and kink-resistant catheter device 100 for vascular applications which has a more flexible distal section so that the catheter is capable of navigating highly tortuous areas of the anatomy and increasingly stiffer sections towards the more proximal region of the catheter. The device has tubular polymer segments 210 making up the shaft core and can have one or more helical ribbon segments 120, 130 arranged as coils and extending in a spiral around the outer surface of the tubular segments. The variability in how the ribbon segments are cut and the amount of material left in different regions can control the stiffness changes along the axial length of the catheter. By combining various tubular segments of different durometer beneath the helical segments, another design variable can be used to create transitions and force transmission capabilities not previous possible with less materials.

## Description

### Field of the Invention

The present disclosure generally relates to devices and methods for accessing blood vessels during intravascular medical treatments. More specifically, the present disclosure relates to a catheter having improved flexibility while maintaining axial stiffness.

### Background

Catheters serve a broad range of functions in intravascular medical treatments. Catheters are typically a thin tube manufactured from medical grade materials that can be inserted into a body and can be used to deliver drugs or other devices, perform surgical procedures, remove blockages from vessels, and a variety of other purposes. By modifying the material or adjusting the way a catheter is manufactured, it is possible to tailor different sections of the catheter for particular applications.

There are a number of access challenges that can make it difficult to access a target site. In cases where access involves navigating the aortic arch (such as with coronary or cerebral blockages) the configuration of the arch in some patients makes it difficult to position a guide catheter. Beyond the arch, accessing the neurovascular bed in particular is challenging with conventional technology, as the target vessels are small in diameter, remote relative to the site of insertion, and are highly tortuous. It is not unusual that a catheter will have to navigate windy pathways with multiple loops, where vessel segments can have several extreme bends in quick succession over only a few centimeters of travel. The ever-narrower reaches of the arterial system can have delicate vessels that can easily be damaged by inflexible or high-profile devices.

Catheters for these procedures can be difficult to design in that they must be fairly stiff at the proximal end to maintain pushability and comfortable manipulation for the user, while having the flexibility in more distal portions to endure high flexure strains and progress through loops and increasingly smaller vessel sizes without causing trauma. For these reasons size, kink-resistance, trackability, and flexibility are the key design parameters usually associated with catheters used in these procedures, and managing the transition from softer to stiffer materials and regions is critical to successful patient outcomes.

Several designs and methods have been proposed for getting a catheter to a target site. In one method, the catheter fits over and is slid along a guidewire which is used to gain access to a target site. A thin guidewire, however, almost always has more reach and distal flexibility than the catheter tube. Newer designs have been proposed which utilize various methods to alter the stiffness between the proximal and distal portions of the catheter, such as braids or windings involving wires or bands of other materials. Currently, most of these catheters control transitions from stiffer materials to softer materials by changing the configuration of the braided member (changing the braid PIC count or coil pitch) or by changing the durometer hardness of the surrounding polymer material. Coils of the braided wires or bands used to reinforce or bond the segments are often a continuous metallic super-elastic or stainless steel of very fine size. While the current innovation can utilize this reinforcement method, these materials add cost and complexity. A sufficiently fine size or diameter of the coils or braids can be prone to kink and difficult to manufacture with the consistency needed for a uniform product.

The material used in the reinforcing windings, and the layering of inner and outer liners or tubes around them means these devices are still fairly stiff. Also, there is no indication these devices are of the flexibility of the designs disclosed herein. Additionally, abrupt stiffness or geometric changes can hinder trackability, introduce significant stress concentrations, and potentially increase the likelihood of device kinking or buckling.

The present designs are aimed at providing an improved catheter construction with controlled stiffness transitions using helical ribbons disposed along the axial length to address the above-stated deficiencies.

### Summary

The innovations of this disclosure involve controlling the stiffness along the length of a catheter shaft by cutting polymer segments that make up the shaft into a "helical" or ribbon spiral pattern. The designs achieve the desired stiffness transitions along the shaft by changing the configuration of the ribbon cut pattern. The underlying tubular members can be of compound construction utilizing varying durometer hardness and dimensions. On the outer layer of helical segments, tapering the helix pitch or changing the helix angle and amount of material left on the ribbon is used to control the catheter's changes in stiffness. Novel transitions can also be created by having overlapping and/or interwoven helical segments. Additionally, using the helical ribbon segments on top of the normal extruded tubular segments can create a ribbed effect on the outside surface of the catheter shaft which can reduce the advancement force required to track the catheter in tortuous areas of the vasculature.

Utilizing these technologies, a device for navigating within body lumens can have one or more elongate tubular segments having an outer surface, an inner lumen, and defining a longitudinal axis. The elongate tubular segments can be arranged and abutted as a longitudinal series along the axis. The tubular segments can be made from the same material or different materials, have the same dimensions or different dimensions, and be fused together to create a continuous structural core. At least a first tubular segment of the one or more segments can have a durometer hardness different from a durometer hardness of another tubular segment.

The device can also have one or more helical segments disposed as a plurality of polymeric ribbon coils in a longitudinally extending spiral around the outer surface of the one or more elongate tubular segments. The helical segments can be adhered to the outer surface using heat or some other suitable method, or they can be formed integrally through machining of a stock shape. Similar to the underlying tubular segments, the helical segments can have the same or differing durometer hardness properties from one another. The dimensions of each helical segment can be tailored to control the stiffness contribution allotted by that segment. The helical segments can also add the necessary body rigidity to the underlying tubular segments to avoid whiplash as the catheter is rotated. In this way, the addition of the ribbon segments can improve force transmission and reduce the number of tubular segments required in construction of the catheter.

In one example, an axial portion of at least one of the helical segments has a spiral width of the ribbon that is different than the spiral width of the ribbon at a different axial portion of the same helical segment. In a separate example, an axial portion of at least one of the helical segments has a helix pitch of the ribbon that is different than the helix pitch of the ribbon at a different axial portion of the same helical segment.

At least one of the one or more helical segments can have a helical pitch which varies continuously along the length of the segment, creating a constantly changing stiffness profile. At least one of the one or more helical segments can overlap axially with a portion of at least one adjacent helical segment, such that multiple helical segments can jointly influence the stiffness along a single axial portion of the elongate tubular segments of the catheter body. In addition to this axial overlap, the helix segments can also overlap radially to create layers outward from the surface of the elongate tubular segments to create very localized positions of enhanced stiffness. The resulting multi-ribbon combinations do not fit flat against the outer surface of the inner core of the one or more elongate tubular segments.

In some cases, the helical segments extend radially outward from the outer surface of the one or more elongate tubular segments. In cases of radial overlap between the helical segments, helical segments can extend radially outward from the outer surface of other helical segments. Depending on the application and desired surface finish, the tubular segments and helical segments can have an outer jacket applied to give the structure a smooth surface to facilitate passage through an outer catheter or the vasculature. In other situations, no outer jacket can be used to give the surface a ribbed finish and feel. Regardless of whether an outer jacket is used, at least a portion of the interior and/or exterior surfaces of the tubular segment and helical segment assembly can be covered with a lubricious low friction coating.

Having a ribbed outer surface on the finished catheter without an added outer jacket can reduce the force required to track the catheter through tortuous anatomy when compared to a conventional design with an outer jacket or hydrophilic coating. These advantages can make the proposed design easier and less expensive to manufacture through the reduction in steps and material processing.

In another example, a catheter tube for navigating body lumens can include an elongate tubular body. The body can have one or more tubular segments of different durometer hardness abutted together in a longitudinal series. These segments can form an outer surface, internal lumen, and constitute the underlying core of the catheter body along the longitudinal axis. In one example, at least one of the one or more tubular segments can have an outer diameter different from another tubular segment so that the elongate tubular body can have a locally or continuously tapered body for enhanced flexibility.

Disposed around the outer surface of the tubular segments of the elongate tubular body can be one or more helical segments. The helical segments can include a plurality of ribbon coils wrapped in a longitudinally extending spiral around the outer surface of the elongate tubular body. The ribbon coils of the one or more helical segments can be fixedly adhered to the outer surface of the elongate tubular body, so they maintain the axial position for which they were intended. The ribbon coils can be wrapped together in a braided fashion or interwoven so that bands of the ribbon which are spiraled in one direction pass over and under bands of another segment spiraled in the opposite direction.

The details of how the helical segments are cut can have a large impact on both the localized and the overall flexibility and rigidity of the catheter. At least one of the one or more helical segments can be cut to have an axial portion along its length with a first spiral width different than a second spiral width of another portion of the same helical segment. Similarly, an axial portion along the length of one of the helical segments can have a first spiral thickness in the radial direction different from a second spiral thickness of another axial portion of the same helical segment.

At least one of the one or more helical segments can axially overlap with at least a portion of at least one adjacent helical segment. The overlapping helical segments can be wound in the same direction or different directions. When wound in the same direction, circumferential edges of adjacent ribbon bands can abut together to form a continuous coil wrap, or an axial gap can be left between adjacent ribbons of the helical segments so that the underlying outer surface of the core of tubular segments is exposed. In still another example, some circumferential edges of the helical segments may abut with edges of an adjacent overlapping helical segment while others can be cut at shallower or steeper angles to diverge from the edges of adjacent segments.

Other processing beyond dimensional aspects can also be used to tailor the stiffness and bending flexibility of the catheter tube. For example, the individual helical segments can have a durometer hardness different from the durometer hardness of other helical segments. In another example, an outer jacket can be reflowed over the structure to bond the helical segments to the outer surface of the elongate tubular body. Additionally, at least a portion of the tubular segment and helical segment assembly can be covered with a lubricious low friction coating.

Other aspects and features of the present disclosure will become apparent to those of ordinary skill in the art, upon reviewing the following detailed description in conjunction with the accompanying figures.

### Brief Description of the Drawings

The above and further aspects of this invention are further discussed with reference to the following description in conjunction with the accompanying drawings. The drawings are not necessarily to scale, emphasis instead being placed upon illustrating the principles of the invention. The figures depict one or more implementations of the inventive devices, by way of example only, not by way of limitation.
Fig. 1 is an isometric view of a catheter with an elongate tubular body as an inner core and polymeric helix segments used to control stiffness along the body according to aspects of the present invention;
Fig. 2A is a representation of a catheter tube having an elongate tubular body configured with a helical ribbon segment according to aspects of the present invention;
Fig. 2B is a cross section of the catheter tube of Fig. 2A according to aspects of the present invention;
Fig. 3 is a representation of an elongate tubular body configured with three overlapping helical ribbon segment according to aspects of the present invention;
Fig. 4 is a view of another catheter tube having helix segments of various pitch and width according to aspects of the present invention;
Fig. 5 shows a cross section of the catheter tube of Fig. 4 according to aspects of the present invention;
Fig. 6 illustrates another catheter tube having a helix segment with a continuously varied pitch according to aspects of the present invention;
Fig. 7 demonstrates an elongate tubular body having compound construction with multiple tubular segments of varying size and properties according to aspects of the present invention;
Fig. 8 is a view of another catheter tube having helix segments of various coil widths and helix angles according to aspects of the present invention;
Fig. 9 shows a cross section of the catheter tube of Fig. 8 according to aspects of the present invention;
Fig. 10 illustrates another catheter tube having helix segments interwoven with various coil widths, thicknesses, and helix angles according to aspects of the present invention; and
Fig. 11 is a cross section view of the catheter tube of Fig. 10 according to aspects of the present invention.

### Detailed Description

The objectives for the designs presented herein can be for a highly flexible and kink-resistant catheter for vascular applications which can have an elongated tubular body section that can be tailored to have different axial polymer segments along its length. The device can have a more flexible distal section so that the catheter is capable of navigating highly tortuous areas of the anatomy, such as the neurovascular, and increasingly stiffer sections towards the more proximal region of the catheter. Disposed on the polymer segments making up the shaft can be one or more helical ribbon segments arranged as coils and extending in spiral around the outer surface of the polymer tube segments. The variability in how the ribbon segments are cut and the amount of material left in different regions controls the stiffness changes along the axial length of the catheter. By combining various axial segments of different durometer beneath the helical ribbon segments, another design degree of freedom can be used to create transitions and force transmission capabilities not previous possible with less materials.

While the description is in many cases in the context of mechanical thrombectomy or other treatments in the neurovascular bed, the devices and methods described may be easily adapted for other procedures and in other body passageways where a catheter with a highly adaptable stiffness requirement is needed. For example, microcatheters typically having a much smaller diameter than other catheters can also be made using these concepts.

Specific examples of the present invention are now described in detail with reference to the Figures, where identical reference numbers indicate elements which are functionally similar or identical. Accessing the various vessels within the vascular, whether they are coronary, pulmonary, or cerebral, involves well-known procedural steps and the use of a number of conventional, commercially available accessory products. These products can involve angiographic materials, rotating hemostasis valves, and guidewires as widely used in laboratory and medical procedures. Though they may not be mentioned specifically by name, when these or similar products are necessarily employed in conjunction with the system and methods of this invention in the description below, their function and exact constitution are not described in detail.

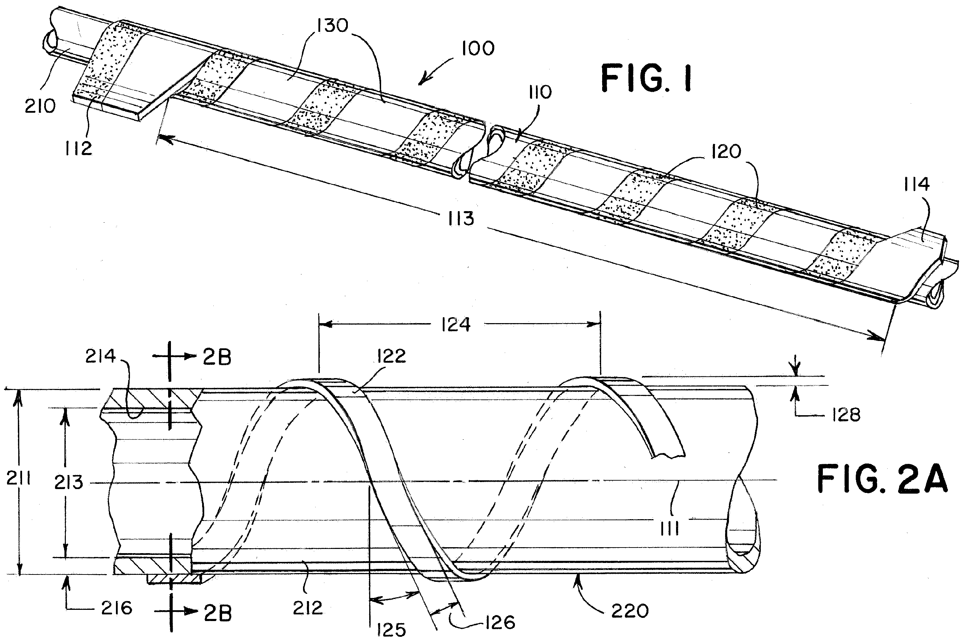

Turning to the figures, in Fig. 1 there is illustrated a catheter shaft device 100 for use in intravascular procedures in the vessels of a patient. The device can combine one or more extruded polymeric tubular segments 210 forming an inner core and one or more spirally wound ribbon-like members forming a helix support structure 110 for controlling the localized stiffness in particular axial portions of the device 100. The helix support structure 110 can be a combination of one or more individual helical segments 120, 130 having a wide variety of design characteristics which can be tuned to adjust the contribution of a particular helix segment to the reinforcement and axial and lateral bending stiffness along a particular longitudinal support length 113 of the device. For example, the spacing, depth, and type of cuts for the helical segments can be varied to control the flexure profile and torsional stiffness of the assembly. The lengths 113 chosen can extend substantially the entire length profile of the elongate tubular body 210 or can be variable and need not be continuous along any portion thereof.

The tubular segments 210 can be made of various medical grade polymers, such as PTFE, polyether block amide (Pebax^{®}), or Nylon. Materials can be chosen, for example, so that more proximal segments are generally harder and less flexible (by durometer hardness, flexure modulus, etc.) as the proximal end 112 is approached. The tubular segments may or may not have a metallic reinforcement layer embedded, such as a braids or ribbons of readily available stainless steels (304SS, 318SS, etc.). With the disclosed design, the choice of material as well as changes to the cut patterns of the helical segments 120, 130 can allow for a level of versatility to be achieved such that a metallic reinforcement layer may not be necessary. Omission of this metallic layer can lead to more simplistic and less expensive catheter construction, where tailoring of the helical segments 120, 130 can provide a sufficient strength-to-weight ratio to be preferable. Furthermore, under tension, braids can tend to lengthen and reduce in cross-section diameter, while under compression, braids can expand in diameter and shorten, reducing the effectiveness of the desired stiffness transitions. Helical segments cut like ribbons and bonded to the outer surface as a reinforcing layer will not have the ability to shorten or lengthen. Additionally, the bonding of the helical segments will aid in supporting the catheter body while under aspiration and other forces.

In another example, a proximal section of the shaft can be cut from variants of high modulus polymer tubes and be joined to a distal section cut from a much more compliant polymer in order to reduce overall cost while affording the benefits of a lower modulus material to the distal end of the device where it is required for enhanced resilience to tight bending curves.

A polymeric cover or membrane (not shown) can also be disposed around at least part of the support framework and tip to enclose the catheter body. In another example, the cover can be a series of polymeric jackets having variable stiffness and flexure properties. The cover can be reflowed, adhered, and/or stitched to the framework of the tubular segment/helical segment structure. Suitable membrane materials can include elastic polyurethanes such as ChronoPrene^{®}, which can have a shore hardness of 40A or lower, or silicone elastomers. A single or variable stiffness cover can also be extruded or post-formed over the catheter tube 100. In other variants, the cover can be laminated, or heat welded to the structure.

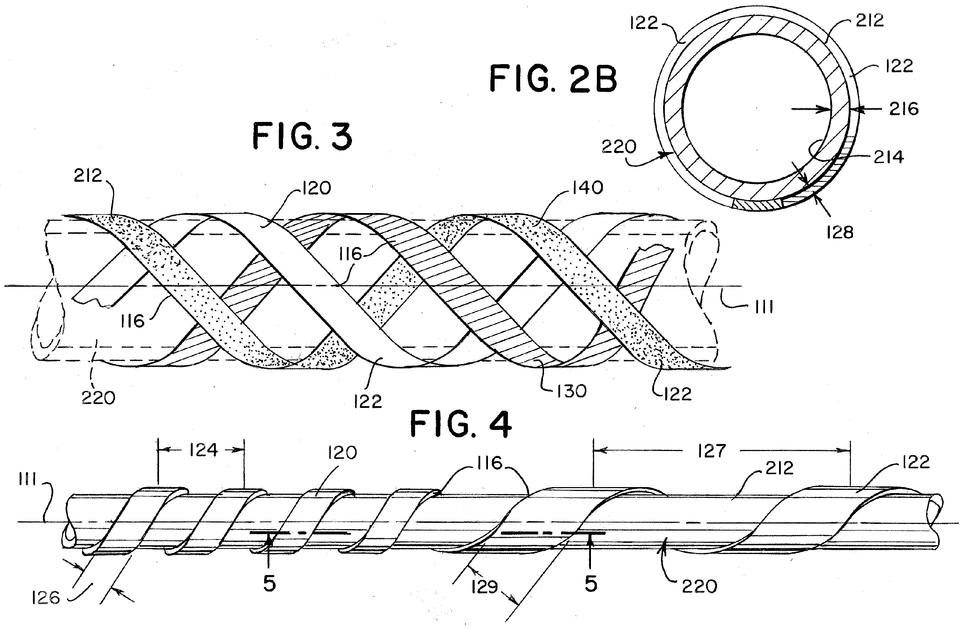

A simplified view showing a reduced layout having a single tubular segment 220 and a single overlaying helical segment 120 is shown in Fig. 2A and in cross section in Fig. 2B. The disposition and orientation of the single tubular segment 220 and single helical segment 120 shown is by demonstration only and not in way of limitation. The tubular segment 220 is shown to be a single, continuous section, but in situations where multiple tubular segments are used individual segments do not need to be continuous, and shorter or longer individual tubular segments can be used in portions where different stiffness or trackability characteristics are preferred.

The helical segment 120 can be broken into individual ribbon coils 122 representing one revolution of the helical segment 120 around the longitudinal axis 111 of the tubular segment 220. Dimensions and material properties the ribbon coils 122 of the helical segment 120 or segments can be utilized to adjust parameters of the device 100 along a selected length. The helix pitch 124, for example, of the windings of the helical segment 120 may be varied to provide for optimum flexibility.

It can be appreciated that the use of different helix segments 120, 130 in the helix support structure 110 to tailor the stiffness and reinforcement can permit the wall thickness 216 of the underlying tubular segments 220 of the catheter to be thinner in some areas with no diminution of performance in areas such as flexibility and crush resistance.

The helix support structure 110 can be formed integrally with or affixed to the outer surface of the elongate tubular body 210 of the device 100 to fix the axial location of the various design features used by the helical segment 120 or segments therein. Heat-shrink, reflowed polymer, and/or adhesives may be used to reinforce the connection between the helix segment 120 or segments of the helix support structure 110 and the tubular body 210.

The described construction technique also allows the production of catheters having small outer diameters which are highly flexible and kink resistant. The flexibility of the resultant device 100 can enable a physician to use a smaller diameter standard sheath or outer access catheter (not shown) to rapidly create a path and gain access to the vicinity of a target site.

To build upon these concepts, Fig. 3 illustrates three helical segments 120, 130, and 140 interwoven on the outer surface 212 of a tubular segment 220. It should be noted that the disposition of the helical segments 120, 130, 140 as shown in Fig. 3 is by example only and is not meant in limitation. As shown, the helical segments can be wrapped or coiled in a way to where there is axial overlap between the segments but oriented so the inside surface of the ribbon coils 122 of each segment can be flush with the outer surface 212 of tubular segment 220. The spacing, or pitch, of the ribbon coils 122 of each segment can be the same or different. The spacing can also be controlled so gaps are arranged between coils of adjacent helical segments that are as dense or porous as needed for the desired level of stiffness. Additional helical segments will have minimal effect on the tubular segment's ability to twist, which can change the preferred bending planes so that the assembly can be capable of self-adjusting as it is advanced through tortuous vessels.

As shown, the ribbon coils 122 of the helical segments 120, 130, 140 need not be fully continuous along the axial length of the tubular segments 220 such that some portions of the outer surface 212 of the tubular segments are visible and without reinforcement. It can also be appreciated that in situations where there are multiple helical segments overlapping axially that some segments may continue along a greater length of the tubular segments 220 beyond the termination points of other helical segments.

In an alternative example envisioned but not illustrated, ribbon coils 122 of the helical segments can be cut longitudinally at specific clocking locations to include interruptions which can be aligned in an alternating pattern such that they form an axial spine or spines. In one instance, the longitudinal cuts can be spaced 180 degrees apart to form two opposing spines parallel to the longitudinal axis 111. Spines spaced 180 degrees apart can define a preferred bending plane for the device 100 running through the spines. Similarly, four longitudinal cuts spaced 90 degrees apart can bias bending in two perpendicular bending planes which are aligned axially to extend through each of the series of cuts. The use of spines can also aid in delivering a balanced and consistent push or thrust force through the length of the catheter tube 100.

Fig. 4 shows an example of a section of a tubular segment 220 which has a helical segment 120 wrapped around the outer surface 212 to provide the segment with specific flexibility advantages. The helical segment 120 is cut so that the helix pitch and coil width are varied between the proximal end 112 and distal end 114 of the assembly. For instance, a first helix pitch 124 can be narrowed or shortened to provide better trackability and torque response near the proximal end 112 of the assembly. Similarly, near the distal end 114 of the assembly where flexibility is more of a concern, the helical segment 120 can have the spacing between turns transition to a second helix pitch 127 is increased over the first helix pitch 124 to better optimize those physical capabilities.

Likewise, in Fig. 4, helical segment 120 can have a second coil width 129 near the distal end 114 that is wider than a first coil width 126 near the proximal end 112. As a result, a high level of variability can be obtained through cutting the wraps of just a single helical segment where the pitch and coil width can transition down the length of the helical segment. Parameters such as pitch and coil width can also be continuously tapered between the proximal end 112 and distal end 114 of the helical segment 120 to avoid abrupt transitions of stiffness.

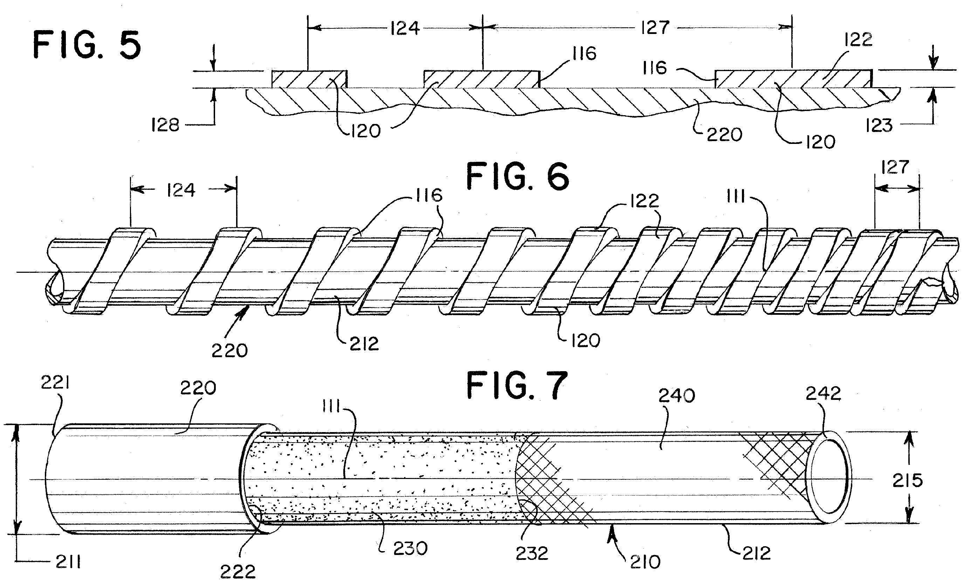

A cross section view through the surface of the tubular segment 220 and helical segment 120 construction of Fig. 4 is pictured in Fig. 5. The ribbon coils of the helical segment 120 can vary in spacing through the first helix pitch 124 and second helix pitch 127. A smaller pitch will generally mean a denser spacing of coils, and thus more reinforcement and stiffness given to the catheter tube 100 in that localized region. When viewed in cross section, each ribbon coil 122 in the wrap of the helical segment 120 will have a proximal and distal circumferential edge 116. If the helix pitch is large enough, gaps between the circumferential edges 116 of adjacent ribbon coils 122 will result in the outer surface 212 of the tubular segment 220 being exposed between the coils. This can be beneficial in certain areas where reinforcement for the underlying tubular segments is not necessary.

An additional design variable can be the thickness of the material in the ribbon coils 122. As illustrated, the ribbon coils 122 can extend radially outward from the outer surface of the tubular segment 220 to form a ribbed or uneven finish on the exterior of the assembly. The thickness 128 of the coils 122 of one helical segment can be different from the thickness cut for another helical segment. Another option can be to cut the helical segment 120 so that a first coil thickness 128 of a more proximal ribbon coil can be greater or less than a second coil thickness 123 of a more distal ribbon coil. In most cases a greater thickness will add more net material or higher modulus material (in situations where material properties are specifically selected for the helical segment) resulting in a localized increase in stiffness for that portion of the assembly of the catheter tube 100.

Fig. 6 illustrates a similar example where a tubular segment 220 is illustrated with a helical segment 120 ribbon cut and coiled around the outer surface 212. The helical segment 120 can have a first helix pitch 124 different from a second helix pitch 127 at a different axial portion of the segment. As an example, the helical segment can be made of a specific polymeric composition (e.g. a polyamide) with mild durometer characteristics and the helix pitch can be continuously varied by a small amount between a proximal end 112 and a distal end 114 in order to add consistent modifying factor to the stiffness throughout the length of the assembly. As a result, the circumferential edges 116 of adjacent ribbon coils 122 of the helical segment 120 get closer together or further apart by a small but constant percentage with each successive coil. A continuously varied pitch will result in the most gradual stiffness transition along the length and provide torsional rigidity while preventing the formation of kink points which can otherwise form at transitions with a higher stiffness gradient. This configuration can also aid in delivering a balanced and consistent push or thrust force through the length of the catheter tube 100.

Multiple tubular segments can be formed in an axial series to make up the elongate tubular body 210. Fig. 7 shows at least a section of an elongate tubular body 210 with three tubular segments, 220, 230, and 240 respectively, of decreasing stiffness distally before any helical segments have been overlaid. The segments can be bonded together at the respective distal ends 222, 232 of tubular segments 220 and 230 so that all segments are concentric with respect to longitudinal axis 111.

The size and length of the segments can be tailored for the specific region of the vasculature for which that segment is intended to operate. For example, a tubular segment 220 near the proximal end 221 of the elongate tubular body can have a first outer diameter 211 and an inner diameter suited to give a wall thickness 216 carefully designed for region to which it is placed. A tubular segment 240 more near the distal end 242 of tubular body 210 which is expected to reside in vessels of smaller size than those proximally can then have a second outer diameter 215 smaller than that that of the first outer diameter 211. As expected, the wall thickness for segment 240 can be carefully designed for this environment and can therefore be the same or different than wall thickness 216 of the more proximal tubular segment 220. It can be appreciated that the specific dimensions of tubular segments 220, 230, and 240 must also be selected so that the catheter tube 100 meets the critical bend criteria as determined for the application.

Material selection is also an important design factor. General selections, such as PTFE and Pebax^{®} have been mentioned previously, but much more specialized materials or blends can be incorporated into specific axial sections of the elongate tubular body 210. In more proximal sections of the catheter where axial stiffness a resistance to collapse are important, the tubular segments 220, 230 can be made from a suitable robust polymer such as polyimide, Nylon, polypropylene, or other materials with a higher density. For more distal sections where flexibility is required, the tubular segment 240 can be for instance a polyurethane, PVC, low density polyethylene (LDPE), or other polymers of suitable modulus and softness. Blends, co-extrusions, and/or mixtures of these and other materials can also be used to obtain the right material properties for a particular segment.

The combination of multiple helical segments with underlying tubular structure has been shown with prototypes to have allowed better stiffness transition and force transmission on a catheter design with only three to five tubular segments than that achieved with a catheter that used to have 12 or more segments, thus significantly simplifying manufacturing and construction. Physician evaluation in *in vitro* models has shown that a catheter with ribbon segments can navigate more distally in the anatomy than a similar catheter with the same durometer polymer segments not cut into ribbons.

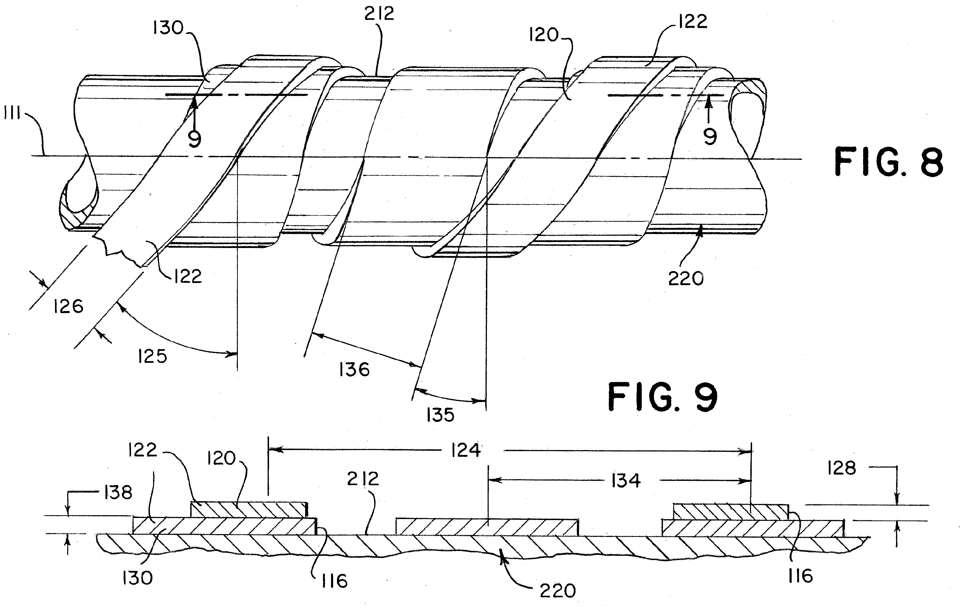

In some examples, the catheter tube 100 can have one or more helical segments which overlap with one another around the outer surface 212 of a tubular segment 220 to form a compound stiffening aspect. Referring to Fig. 8, a section of a tubular segment 220 has a first helical segment 120 is combined with a second helical segment 130. During processing, the second helical segment 130 can be wrapped as a ribbon around the tubular segment 220 first and the position of its ribbon coils 122 set to give a desired coil width 136 and helix angle 135 with respect to the longitudinal axis 111. Once the second helical segment 130 is in position, the assembly can be overlaid with the first helical segment 120 so that it overlaps radially and longitudinally with the second helix segment and tubular segment. The first helical segment 120 can have a coil width 126 and helix angle 125 the same of different than that used for the second helical segment. In this variant of the catheter tube 100, the outer layer of the assembly can be either the tubular segment 220, first helical segment 120, or second helical segment 130 depending on what discrete axial position along the outer surface 212 of the elongate tubular body 210 is referenced.

As with previous examples, the outer surface of the assembly can be left with the ribbed finish achieved by the winding of helical segments 120 and 130, or an outer polymer jacket or cover can be reflowed in place for a smooth surface. In an alternate example, a thin shrink-wrap layer of polyethylene or similar material can be used to further pull the coils of the helical segments to the surface of the tubular member to adjust the surface asperities of the otherwise ribbed finish.

A cross section through the wall thickness of the tubular segment 220 from the same example of the catheter tube 100 is illustrated in Fig. 9. The greater magnitude of the helix coil pitch 124 of the first helical segment 120 than that of the second helical segment 130 can mean that some but not all of the ribbon coils 122 from the second helical segment 130 will be overlaid with those from the first. Alternately, if the pitch 134 of the second helical member 130 were to be increased to a great extent, they can be axial positions along the length of the tubular segment 220 where ribbon coils 122 from the first helical segment will fall in a gap between the ribbon coils 122 of the second helical segment and therefore be bonded to the outer surface of the tubular segment as the only reinforcing layer at that position.

As with other examples, the coil thickness of the individual ribbon coils 122 of the helical segments can be cut to vary the radial thickness 128, 138 of the helical segments. The thicknesses and overlap of the coils can be chosen such that the overall radial size of the catheter body will still fit within standard guide sheaths and intermediate catheters.

In addition, it is evident that similar to other examples previously discussed, the illustrated wrapping of helical segments 120 and 130 on top of the normal extrusion tubular segments 220 will result in an outer surface of the assembly that is uneven or ribbed. In order to allow for smooth delivery of the catheter through an outer catheter, the outer surface is often coated with a low-friction or lubricious material, such as PTFE or FEP. However, we have found that when testing such construction without any lubricious hydrophilic coating in a tortuous model that they tracked further and with less force than a conventional construction catheter with various axial durometer segments which had a lubricious hydrophilic coating. It is therefore conceivable that the use of polymeric helical ribbon construction might allow for the removal of hydrophilic coatings as necessary for such catheters. At a minimum, such coatings may not need to be as lubricious as is needed in current designs.

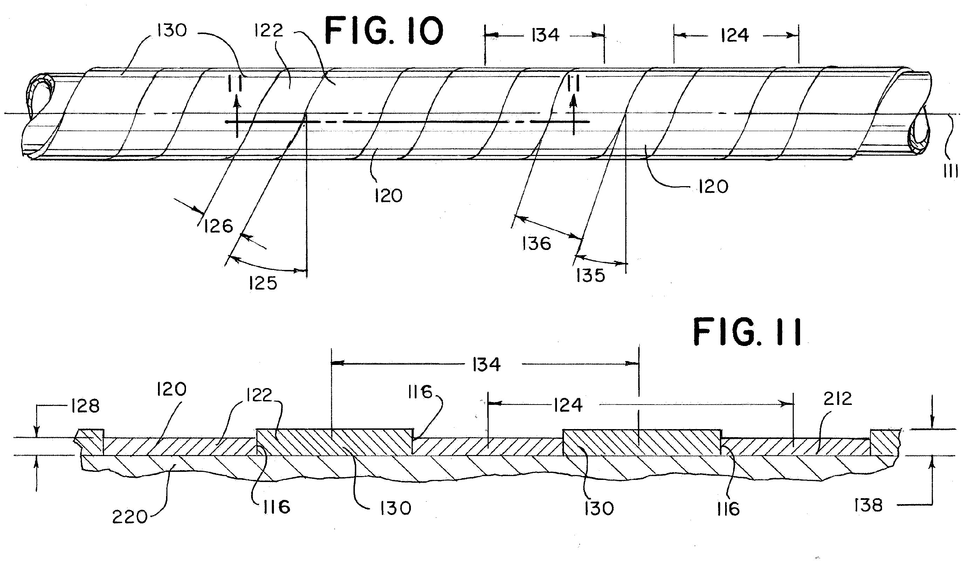

Another example of one or more helical segments which overlap with one another around the outer surface 212 of a tubular segment 220 is shown in Fig. 10. A cross sectional view of the same arrangement is shown in Fig. 11. In this example, the helix pitch 124 and helix angle 125 of the first helical segment 120 are selected such that the pitch is constant, and the helix angle matches the helix angle 135 of the second helical segment 130. In this way the circumferential edges 116 of the coils abut to form a continuous reinforcing layer along the length of the segment. The actual stiffness contribution from such an arrangement can have an amplitude that is more of a sinusoidal pattern, where the capabilities of the underlying tubular segment 220 are influenced at regularly alternating axial positions by the dimensional and material property differences between the first helical segment 120 and the second helical segment 130. Having interwoven helical segments 120, 130 with abutting circumferential edges 116 also allows for stiffness transitions to be blended along the length of the tube without regular gaps where stress concentrations may otherwise be prone to kink the tube.

As seen in the cross-sectional view in Fig. 11, the helix pitch 124, 134 of the helical segments can be varied so that the alternating pattern allows a desired alignment of properties to be reached. A first coil thickness 128 of the first helical segment 120 at a more proximal location can be greater than a second coil thickness 138 of the second helical segment 130 at a more distal location. In general, less material in the ribbon helical segments will result in a more flexible section of the catheter tube. Similarly, the coil thicknesses 128, 138 can be varied along the length of the helical segments 120, 130 so that a more custom profile can be created.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. It is intended that each term contemplates its broadest meaning as understood by those skilled in the art and includes all technical equivalents that operate in a similar manner to accomplish a similar purpose without departing from the scope and spirit of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology. For clarity and conciseness, not all possible combinations have been listed, and such variants are often apparent to those of skill in the art and are intended to be within the scope of the claims which follow.

## Claims

1. A device for navigating within body lumens, the device comprising:
one or more elongate tubular segments comprising an outer surface, an internal lumen, and a longitudinal axis, the elongate tubular segments disposed in a longitudinal series along the longitudinal axis; and
one or more helical segments disposed as a plurality of polymeric ribbon coils configured in a longitudinally extending spiral around the outer surface of the one or more elongate tubular segments;
a first tubular segment of the at least one of the one or more elongate tubular segments comprising a first durometer hardness different from a second durometer hardness of another tubular segment;
the one or more helical segments being fixedly adhered to the outer surface of the elongate tubular segments.

2. The device of claim 1, wherein at least one of the one or more helical segments comprises an axial portion with i) a spiral width different than the spiral width of another axial portion of the same helical segment or ii) a helix pitch different than the helix pitch of another axial portion of the same helical segment.

3. The device of claim 1, wherein at least one of the one or more helical segments comprises a helical pitch which varies continuously along the length of the segment.

4. The device of claim 1, wherein at least one of the one or more helical segments axially overlaps with a portion of at least one adjacent helical segment, optionally wherein at least a portion of the overlapping adjacent helical segments overlap radially.

5. The device of claim 1, wherein the elongate tubular segments are configured to be integral with the helical segments.

6. The device of claim 1, wherein a first helical segment comprises a durometer hardness different from a second helical segment.

7. The device of claim 1, wherein the ribbon coils of the helical segments extend radially outward of the outer surface of the one or more elongate tubular segments.

8. The device of claim 1, wherein a lubricious hydrophilic coating is disposed around at least a portion of the elongate tubular segments and helical segment.

9. A catheter tube for navigating within body lumens, the catheter tube comprising:
an elongate tubular body comprising one or more tubular segments of differing durometer hardness, the tubular segments abutted in a longitudinal series along a longitudinal axis forming an outer surface and an internal lumen of the elongate tubular body; and
one or more helical segments comprising a continuous plurality of ribbon coils configured in a longitudinally extending spiral around the outer surface of the elongate tubular body.

10. The catheter tube of claim 9, the one or more helical segments being fixedly adhered to the outer surface of the elongate tubular body.

11. The catheter tube of claim 9, wherein at least one of the one or more tubular segments comprises a diameter different from another tubular segment.

12. The catheter tube of claim 9, wherein at least one of the one or more helical segments comprises an axial portion with a first spiral width different than a second spiral width of another axial portion of the same helical segment.

13. The catheter tube of claim 9, wherein at least one of the one or more helical segments comprises an axial portion with i) a first spiral thickness different than a second spiral thickness of another axial portion of the same helical segment or ii) a first helical pitch different than a second helical pitch of another axial portion of the same helical segment.

14. The catheter tube of claim 9, wherein at least one of the one or more helical segments axially overlaps with a portion of at least one adjacent helical segment, optionally wherein at least a portion of the ribbon coils of the axially overlapping adjacent helical segments abut at one or more circumferential edges.

15. The catheter tube of claim 9, wherein at least one of the one or more helical segments comprises a durometer hardness different from another helical segment.

16. The catheter tube of claim 9, wherein a lubricious hydrophilic coating is disposed around at least a portion of the elongate tubular body and helical segments.
